# EUROPEAN PATENT SPECIFICATION

(11) **EP 1 818 003 B1**
(45) Date of publication and mention of the grant of the patent: **19.08.2015**
(21) Application number: 05805393.5
(22) Date of filing: 31.10.2005
(51) Int. Cl.: A61B 1/04, A61B 1/06, A61B 1/00

(54) **BODY INSERTABLE APPARATUS**
IN DEN KÖRPER EINFÜHRBARES GERÄT
DISPOSITIF INTRODUIT DANS UN SUJET

(30) Priority: 29.11.2004 JP 2004344953
(43) Date of publication of application: 15.08.2007
(73) Proprietor: Olympus Corporation, Tokyo 151-0072 (JP)
(72) Inventor: FUJIMORI, Noriyuki, Tokyo 151-0072 (JP)
(74) Representative: von Hellfeld, Axel
(86) International application number: PCT/JP2005/020006
(87) International publication number: WO 2006/057136

(56) References cited:
- EP-A2- 1 342 447
- WO-A2-01/50941
- WO-A2-03/009739
- JP-A- 2003 210 393
- JP-A- 2003 260 025
- JP-A- 2005 074 031
- JP-A- 2005 205 078
- US-A1- 2004 027 459
- US-A1- 2004 215 059

## Description

### TECHNICAL FIELD

The present invention relates to a body insertable apparatus such as a swallowable capsule endoscope that includes various function execution units for executing a predetermined function of collecting information on an inside of the subject into which the capsule endoscope being inserted, and includes a board on which the function execution units are arranged.

### BACKGROUND ART

Recently, a capsule endoscope in which an imaging function and a radio function are installed has been proposed in a field of an endoscope. The capsule endoscope is configured to travel inside organs (inside of a body cavity) such as a stomach or a small intestine by a peristaltic movement to capture images one by one by using the imaging function, during an observation period from when the capsule endoscope is swallowed by an examinee as a subject (human body) for an observation (examination) until the capsule endoscope is naturally excreted from a body of the examinee.

Image data captured inside the body cavity by the capsule endoscope during the observation period of traveling inside the organs is sequentially transmitted to an external device provided outside the subject, through the radio function such as a Bluetooth, and stored in a memory provided in the external device. By carrying the external device including the radio function and a memory function, the examinee can move without inconvenience during the observation period from when the examinee swallows the capsule endoscope until the capsule endoscope is excreted. After the observation is finished, a doctor or a nurse makes a diagnosis by displaying the images of the body cavity on a display unit such as a display, based on the image data stored in the memory of the external device.

The above type of the capsule endoscope includes a swallowable type of the capsule endoscope disclosed in Patent Document 1, for performing the above functions. Such capsule endoscope has been proposed that includes an illuminating unit (light emitting diode; hereinafter, "LED"), an image sensor, a driving circuit, a power unit including, i.e., a battery, and a transmitting unit for transmitting image data from the image sensor to the external device, each of which is arranged on each of arrangement boards with, for example, an integrated circuit (IC) structure. The boards are connected by a strip board and parts are installed in a capsule-shaped closed container that has dome-shaped tip portions.

Patent Document 1: International application No. 02/102224 pamphlet

### DISCLOSURE OF INVENTION

### PROBLEM TO BE SOLVED BY THE INVENTION

With the above capsule endoscope, a plurality of the LEDs is arranged in a dome-shaped front-tip cover on a front tip portion, and the LEDs output an illuminating light for illuminating an inside of the subject through the transparent front-tip cover. The LED is configured in a rectangular solid that is, for example, 1.6 mm wide, 0.8 mm long, and 0.6 mm high, and includes two electrodes formed on a bottom portion and an irradiation unit formed on a top portion of the power unit to output the illuminating light. The LEDs are arranged in the periphery of the image sensor; however, there is no enough space for the front-tip cover or a light for capturing an image. To obtain the enough space, the capsule endoscope is required to be large, and therefore, it is difficult to realize a downsizing. Further, if the LEDs are arranged close to the image sensor, the LEDs come in a field of view determined by an optical quality, such as an aperture or a focal length, of a lens. As a result, such problem occurs that an optical flare is generated on a captured image.

The present invention has been made in view of the above problems. An object of the present invention is to provide a body insertable apparatus that can prevent the optical flare from being generated and realize a downsizing of the capsule endoscope.

### MEANS FOR SOLVING PROBLEM

To solve the above problems and to achieve the above objects, according to the present invention, a body insertable apparatus includes an outer case having at least one dome-shaped end portion; an imaging unit, arranged in the outer case, that captures an image of an inside of a body into which the body insertable apparatus being inserted, and acquires an image information of the inside of the body; an illuminating unit that includes an electrode unit provided in the dome-shaped end portion and in the periphery of the imaging unit, and an irradiation unit formed on a top portion of the electrode unit, an end surface of the irradiation unit in a longitudinal direction being constituted to be shorter than an end surface of the electrode unit, the irradiation unit providing a plurality of light emitting diodes that outputs an illuminating light for illuminating the inside of the body from which an image is captured by the imaging unit; and an arrangement board, provided in the outer case, on which the imaging unit and the illuminating unit are arranged respectively.

In the body insertable apparatus according to the invention, each of the light emitting diodes may be arranged so that a longitudinal side of the light emitting diode is arranged along a circumferential direction with a predetermined radius having the imaging unit as a center.

In the body insertable apparatus according to the invention, each of the light emitting diodes may be arranged so that a longitudinal side of the light emitting diode is arranged along a radial direction with a predetermined radius having the imaging unit as a center.

In the body insertable apparatus according to the invention, each of the light emitting diodes may be arranged so that a longitudinal side of the light emitting diode is arranged being inclined from a radial direction with a predetermined radius having the imaging unit as a center.

In the body insertable apparatus according to the invention, the predetermined radius having the imaging unit as a center, may be set larger than a field of view determined by an optical quality of an optical system of the imaging unit.

### EFFECT OF THE INVENTION

A body insertable apparatus according to the present invention enables to prevent the optical flare from being generated and to realize a downsizing of the capsule endoscope, by arranging in the periphery of an imaging unit in a dome-shaped front-tip cover of a tip portion, a plurality of LEDs including a power unit connected to an arrangement board and an irradiation unit arranged on a top portion of the power unit, with an end surface having a longitudinal side being configured to be shorter than an end surface of the power unit, and by outputting an illuminating light by the irradiation unit for illuminating inside of the subject to be captured into an image by the imaging unit.

### BRIEF DESCRIPTION OF DRAWINGS

FIG. 1 is a schematic view of an entire configuration of a wireless in-vivo information acquiring system that includes a body insertable apparatus according to an embodiment of the present invention;
FIG. 2 is a sectional side view of a configuration of the body insertable apparatus according to the embodiment of the present invention;
FIG. 3 is a top view of an expanded rigid/flexible-printed circuit board shown in FIG. 2;
FIG. 4 is a sectional view of a front surface of an illuminating board shown in FIG. 2, according to a first embodiment of the present invention;
FIG. 5 is a side view of a light emitting diode (LED) shown in FIG. 3;
FIG. 6 is a sectional view of a rear surface of a transmitting board shown in FIG. 2;
FIG. 7 is a sectional view of a front surface of the illuminating board shown in FIG. 2, according to a second embodiment of the present invention;
FIG. 8 is a sectional side view of the periphery of a front-tip cover of the body insertable apparatus shown in FIG. 2; and
FIG. 9 is a sectional view of a front surface of the illuminating board shown in FIG. 2, according to a third embodiment of the present invention.

### EXPLANATIONS OF LETTERS OR NUMERALS

- 1: Subject
- 2: Receiving device
- 2a: Receiving jacket
- 2b: External device
- 3: Capsule endoscope
- 4: Display device
- 5: Portable recording medium
- 6: Closed container
- 7: Illuminating unit
- 8: Imaging unit
- 9: Control unit
- 10: Accumulator unit
- 11: Switching board (rigid board)
- 12: Power board (rigid board)
- 13: Button type battery
- 14: Reed switch
- 15: Power control IC
- 16: Switching unit
- 17: Contact
- 18: Power unit
- 19: Regulator
- 20: Radio transmitter
- 21: Transmitting board (rigid board)
- 22: Oscillating circuit
- 23: Antenna
- 24: Connecting terminal
- 31: Flexible board
- 32: Rigid/flexible-printed circuit board
- 61: Front-tip cover
- 62: Body cover
- 63: Body
- 64: Rear tip portion
- 65, 66: Bonding tip portion
- 67a, 66a: Bonding surface
- 65b: Projection
- 66b: Groove
- 71: Illuminating board (rigid board)
- 71a: Insertion hole
- 72: Light emitter (LED)
- 72a: Electrode
- 72a1, 72b1,: End surface
- 72b: Irradiation unit
- 72c: Medial angle region
- 72d: Lateral angle region
- 74, 85, 92: Chip component
- 81: Imaging board (rigid board)
- 82: Solid-state image sensor
- 83: Imaging lens
- 83a, 83b: lens
- 84: Focus adjustment mechanism
- 84a: Movable frame
- 84b: Fixed frame
- A1: to An Receiving antenna

### BEST MODE(S) FOR CARRYING OUT THE INVENTION

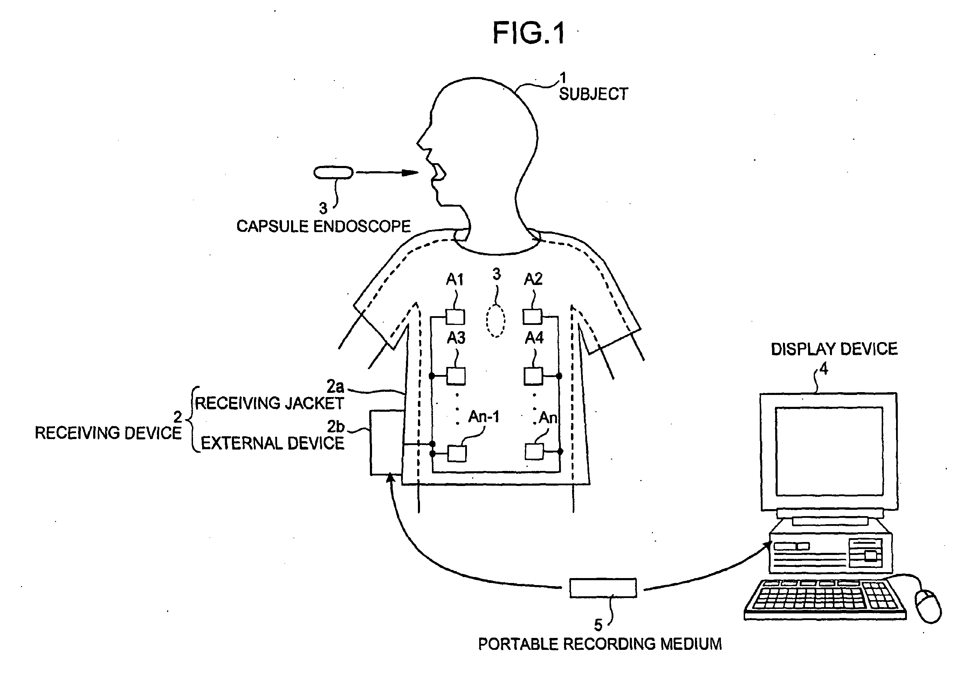

FIG. 1 is a schematic view of an entire configuration of a wireless in-vivo information acquiring system that includes the body insertable apparatus according to an embodiment of the present invention. With the in-vivo information acquiring system, a capsule endoscope that is inserted from, for example, a human mouth of a subject into a body cavity for capturing an image of an examined region of the body cavity will be described as an example of the body insertable apparatus. In FIG. 1, the in-vivo information acquiring system includes a receiving device 2 that includes a radio receiving function, and a capsule endoscope 3 that is inserted into a subject 1 to capture a body cavity image and transmits data such as an image signal to the receiving device 2. The in-vivo information acquiring system further includes a display device 4 that displays the body cavity image based on the image signal received by the receiving device 2, and a portable recording medium 5 for transmitting and receiving the data between the receiving device 2 and the display device 4.

The receiving device 2 includes a receiving jacket 2a to be worn by the subject 1 and an external device 2b that performs a process for a received radio signal, both of which are fixed on the subject 1, for example, on a lumber portion of the subject 1 with a not shown belt, to be taken along by the subject 1. In other words, the receiving device 2-includes a function of receiving image data of the body cavity transmitted by radio from the capsule endoscope 3, the receiving jacket 2a includes receiving antennas A1 to An and is arranged in a wearable form, and the external device 2b performs a process of the radio signal received via the receiving antennas A1 to An in the receiving jacket 2a.

The display device 4 is to display, for example, the body cavity image captured by the capsule endoscope 3 and has a configuration of, for example, a workstation that displays an image based on data acquired by the portable recording medium 5. Specifically, the display device 4 can be configured for displaying an image directly on a cathode-lay tube (CRT) display or on a liquid crystal display, or for outputting the image to other media such as a printer.

The portable recording medium 5 is removable for the external device 2b and the display device 4, and has a configuration for realizing data output and data recording when being inserted into each of the devices. According to the present embodiment, the portable recording medium 5 is inserted into the external device 2b and records data transmitted from the capsule endoscope 3 while the capsule endoscope 3 travels inside the body cavity of the subject 1. When the capsule endoscope 3 is excreted from the subject 1, that is, when imaging of the inside of the subject 1 is finished, the portable recording medium 5 is removed from the external device 2b and inserted into the display device 4, and then, the display device 4 reads data recorded in the portable recording medium 5. For example, if data transfer between the external device 2b and the display device 4 is performed by the portable recording medium 5 configured with, for example, a compact flash memory (registered trademark), the subject 1 can freely move during an operation of capturing the body cavity image, compared to a case in which the external device 2b and the display device 4 are directly connected using a wire. According to the present embodiment, the portable recording medium 5 is used for the data transfer between the external device 2b and the display device 4. However, the configuration is not limited to the above and is possible to be others; for example, other built-in recording media, i.e., a hard disk drive, can be used as the external device 2b and the external device 2b and the display device 4 are connected with a wire or without a wire for the data transfer.

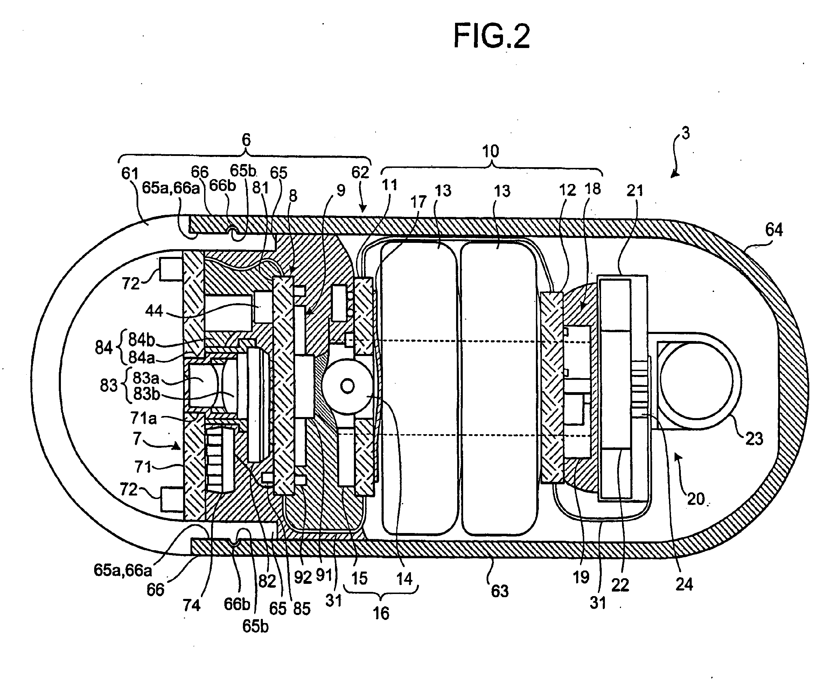

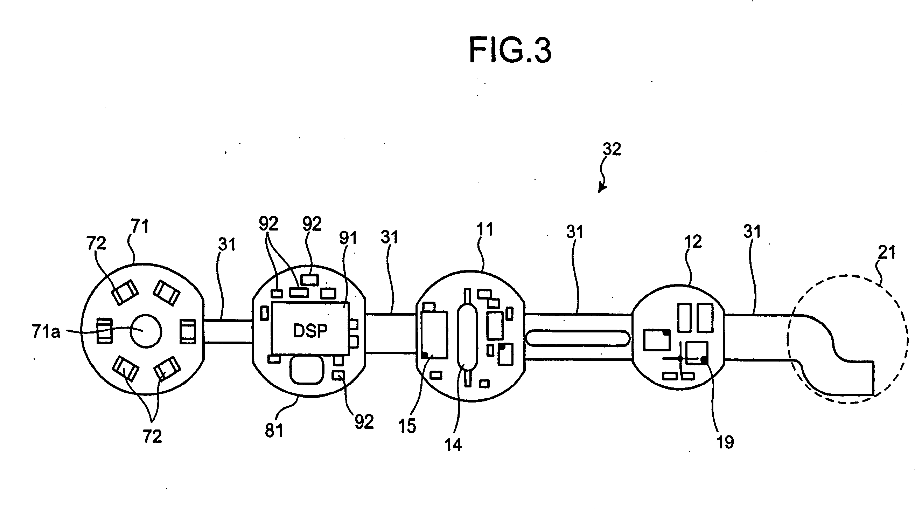

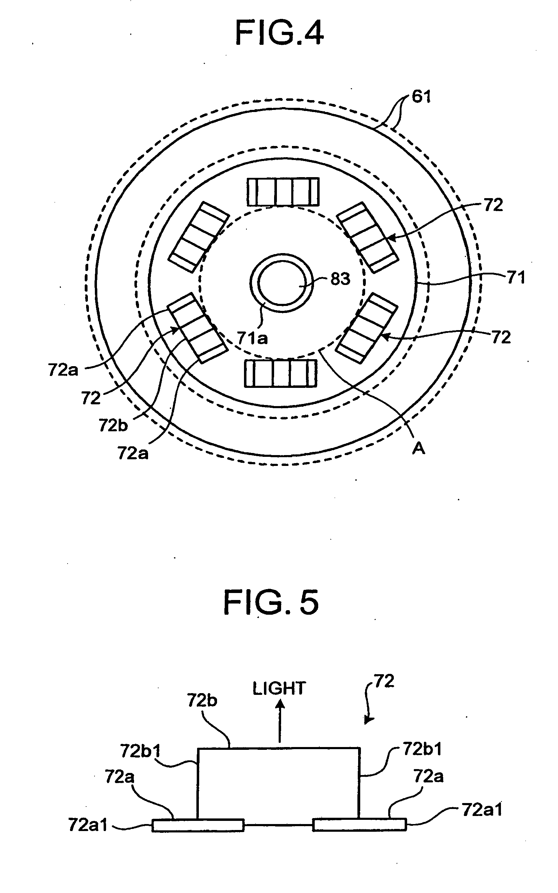

FIG. 2 is a sectional side view of a configuration of the body insertable apparatus (the capsule endoscope 3) according to the embodiment of the present invention. FIG. 3 is a top view of an expanded rigid/flexible-printed circuit board shown in FIG. 2. FIG. 4 is a sectional view of a front surface (a side of a front-tip cover) of an illuminating board shown in FIG. 2. FIG. 5 is a sectional view of a rear surface (a side of a rear tip portion) of a transmitting board shown in FIG. 2.

The capsule endoscope 3 includes, as shown in FIG. 2, a closed container 6 that is an outer case formed in a capsule shape, an illuminating unit 7, as a function execution unit for executing predetermined functions, that outputs an illuminating light for illuminating an examined region in the body cavity, an imaging unit 8, as the function execution unit, that captures an image of the examined region by receiving a reflected light of the illuminating light, a control unit 9 that performs a drive control of the illuminating unit 7 and the imaging unit 8 and performs a signal process, an accumulator unit 10 that stores driving power for driving the function execution units, and a radio transmitting unit 20, as the function execution unit, that transmits by radio, image data acquired by the imaging unit 8 to the outside of the subject.

The closed container 6 is in a size swallowable by a human and formed by elastically engaging a substantially hemisphere-shaped front-tip cover 61 with a cylindrical-shaped body cover 62. As arrangement boards, an illuminating board 71, an imaging board 81, a switching board 11, a power board 12 and a transmitting board 21 are installed in the cylindrical-shaped body cover 62 that has a substantially hemisphere-shaped bottom portion on a rear tip portion and has a circular-shaped front tip portion that is open. The front-tip cover 61 is in a substantially hemisphere dome shape and a rear side of the dome is open in a circular shape. The front-tip cover 61 is made of a transparent material having a transparency or a translucency, such as a cyclo-olefin polymer or a polycarbonate, which is suitable for assuring, for example, an optical quality and strength. The front-tip cover 61 realizes to transmit an illuminating light from the illuminating unit 7 to the outside of the closed container 6 and to transmit the reflected light of the illuminating light from the subject to the inside.

The body cover 62 is arranged on a rear side of the front-tip cover 61 and covers the above function execution units. The body cover 62 integrally forms a cylindrical-shaped body portion 63 and a substantially hemisphere-dome-shaped rear tip portion 64, and a front surface of the body portion 63 is open in a circular shape. The body cover 62 is made of, for example, a polysulphone suitable for assuring strength, and the illuminating unit 7, the imaging unit 8, the control unit 9, and the accumulator unit 10 are installed in the body portion 63 while the radio transmitting unit 20 is installed in the rear tip portion 64.

A cylindrical-shaped bonding tip portion 65 is arranged along the periphery of an opening tip portion of the opening of the front-tip cover 61. Further, a cylindrical-shaped bonding tip portion 66 is arranged along the periphery of an opening tip portion of the opening of the body portion 63. Each of the bonding tip portions 65 and 66 includes each of bonding surfaces 65a and 66a to be in contact with each other and overlapped at the inside and the outside of the closed container 6, when the front-tip cover 61 and the body cover 62 are bonded. According to the present embodiment, the bonding tip portion 65 of the front-tip cover 61 is arranged inside the closed container 6, with an outer surface being configured to be the bonding surface 65a, while the bonding tip portion 66 of the body cover 62 is arranged outside the closed container 6, with an inner surface being configured to be the bonding surface 66a, and an outer diameter of the bonding surface 65a and an inner diameter of the bonding surface 66a are configured in substantially same sizes. Each of the bonding surfaces 65a and 66a is formed with a draft angle of zero degree for a shape forming, and formed in a cylindrical shape having a substantially same inner or outer diameter, to make a bonding easy.

A projection 65b is formed in an endless manner on an entire circumference of the bonding surface 65a, and a groove 66b is formed in an endless manner on an entire circumference of the bonding surface 66a. The projection 65b and the groove 66b are engaged with each other when the bonding surfaces 65a and 66a are overlapped. As described, the projection 65b and the groove 66b structure, by being engaged with each other, a bonding hold unit that holds a bonded state between the front-tip cover 61 and the body cover 62.

The illuminating unit 7 includes, as shown in FIG. 2 to FIG. 5, the disk-shaped illuminating board 71 with a center portion on which an insertion hole 71a is arranged, six light emitters of light emitting diodes 72 such as a white LED arranged on a front surface (a side of the front-tip cover 61 in FIG. 2) of the illuminating board 71, and a chip component 74 that structures a circuit for driving the LED 72, arranged on a rear surface (a side of the imaging board 81 in FIG. 2). The illuminating light from the LED 72 is output to the outside via the front-tip cover 61.

Each of the LEDs 72 has the same configuration including, as shown in FIG. 5, an electrode unit that includes two electrodes 72a connected to the illuminating board 71, and an irradiation unit 72b formed on a top portion of the electrode unit. The irradiation unit 72b is formed in a stair shape with an end surface 72b1 in a longitudinal direction being configured to be shorter than an end surface 72a1 of the electrode unit. With the LED 72, by applying power voltage to the electrodes 72a, the irradiation unit 72b emits light to output the illuminating light from a top surface to the outside.

The LEDs 72 are, as shown in FIG. 4, arranged in the periphery of an imaging lens 83 as an optical system of the imaging unit 8 described later, on the illuminating board 71 in an equally-spaced manner. In other words, each of the LEDs 72 is arranged so that each of the longitudinal side is to be along a direction of a circumference "A" having a radius that is wider than a field of view determined by an optical quality of the imaging lens 83, with the imaging lens 83 of the imaging unit 8a as a center, in an equally-spaced manner.

With the LED 72 having the above configuration, a side surface is formed in a stair shape so that a distance between each of the end surfaces 72a1 of the electrode unit equals 1.6 mm, a distance between each of the end surfaces 72b1 of the irradiation unit 72b equals 1 mm, a longitudinal (a length of the end surfaces 72a1 of the electrode unit or the end surfaces 72b1 of the irradiation unit 72b) equals 0.8 mm, a height (a height including the end surface 72a1 of the electrode unit and the end surface 72b1 of the irradiation unit 72b) equals approximately 0.6 mm. Thus, the LED 72 is formed so that the distance between each of the end surfaces 72b1 of the irradiation unit 72b is configured to be shorter than the distance between each of the end surfaces 72a1 of the electrode unit, compared to an LED with a conventional rectangular-solid configuration. Accordingly, if the LED 72 according to the present embodiment is arranged on the same position of the illuminating board 71 as with the conventional LED, a longer distance is generated between an outer corner portions (a side of the front-tip cover 61) among corner portions on the top surface of the irradiation unit 72b of the LED 72 and an inner surface of the front-tip cover 61, compared to the conventional LED. Therefore, if the LED 72 according to the present embodiment is arranged on a position shifted to a side of the front-tip cover 61, the LED 72 can be arranged on a circumference that is larger than that of the above described field of view, with an enough space. As a result, optical flare can hardly be generated.

If the LED 72 according to the present embodiment is arranged on the same position of the illuminating board 71 as with the conventional LED, a longer distance is generated between the LED 72 and the front-tip cover 61. Therefore, a longer distance than the conventional length is generated, and the illuminating board 71 can be downsized by a corresponding distance. As a result, it is possible to downsize the front-tip cover 61 and to realize a downsizing of the entire capsule endoscope. Further, according to the present embodiment, if the arrangement position of the LED 72 is adjusted within an area corresponding to a distance generated between the LED 72 and the front-tip cover 61, it is possible to realize the downsizing of the front-tip cover 61 and to prevent the optical flare from being generated.

The imaging unit 8 includes, as shown in FIG. 2, the disk-shaped imaging board 81, a solid-state image sensor 82 such as a charge-coupled device (CCD) or a complementary metal-oxide semiconductor (CMOS) arranged on a front surface (a side of the illuminating board 71 in FIG. 2) of the imaging board 81, and the imaging lens 83 that forms an image of a subject on the solid-state image sensor 82. The imaging lens 83 is arranged on a front surface (a side of the illuminating board 71 in FIG. 2) of the solid-state image sensor 82, and includes a first lens 83a and a second lens 83b arranged on a movable frame 84a on a position at a side of the subject. The movable frame 84a and a fixed frame 84b structure a focus adjustment mechanism 84 that shifts the first lens 83a and the second lens 83b along an optical axis. Further, the movable frame 84a is inserted into the insertion hole 71a of the illuminating board 71 and adjusts the optical axis of the imaging lens 83 toward the front surface of the illuminating board 71. Accordingly, the imaging unit 8 can capture an image of a region illuminated by the illuminating light of the illuminating unit 7. On the front surface of the imaging board 81, a chip component 85 that structures a circuit for driving the solid-state image sensor 82 is arranged so that the chip component 85 surounds the solid-state image sensor 82.

The control unit 9, as shown in FIG. 2 and FIG. 3, includes a DSP (digital signal processor) 91 and the DSP is arranged on a rear surface of the imaging board 81 so that the DSP 91 is rounded by a chip component 92. The DSP 91 performs a central role of a driving control of the capsule endoscope 3 and performs a driving control of the solid-state image sensor 82, an output signal process, and a driving control of the illuminating unit 7. The chip component 92 on a rear surface of the imaging board 81 is a semiconductor member that includes a function of mixing two signals including an image signal and a clock signal output from the DSP 91 into a single signal when the signals are transmitted from the radio transmitting unit 20.

The accumulator unit 10 includes, as shown in FIG. 2, a button type battery 13 such as a silver oxide battery, the disk-shaped switching board 11, a reed switch 14, a power control IC 15, a switching unit 16 arranged on a front surface (a side of the imaging board 81 in FIG. 2) of the switching board 11, and a power unit 18. A plurality of the button type batteries 13, for example, two of which, according to the present embodiment, are arranged in series and a side of a negative-electrode cap is toward a rear surface. The batteries 13 are not limited to the silver oxide battery and can be, for example, a rechargeable battery and a generator battery, and the number is neither limited to two. A contact 17 is formed by a blade spring on a rear surface of the switching board 11 and comes contact with a positive-electrode-can of the button type battery 13 to energize the button type battery 13 toward a rear surface (a side of the power board 12 in FIG. 2) by energizing power of the blade spring.

The power unit 18 includes the disk-shaped power board 12 and a regulator 19 arranged on a rear surface (a side of the rear tip portion 64 in FIG. 2) of the power board 12. The regulator 19 performs a control of, for example, stepping down a voltage acquired by the button type battery 13 to constantly acquire a predetermined voltage necessary for the system. Further, although it is not shown in the drawings, a contact that comes contact with a negative-electrode cap of the button type battery 13 is arranged on a front surface (a side of the switching board 11 in FIG. 2) of the power board 12. According to the present embodiment, the accumulator unit 10 realizes a power supply for each of the function execution units, by connecting and arranging the button type batteries 13 in series between the switching board 11 and the power board 12.

The radio transmitting unit 20 is formed in a cylindrical shape and includes the transmitting board 21 having an internal space area, an oscillating circuit 22 arranged inside the transmitting board 21, an antenna 23 arranged on a rear surface (a side of the rear tip portion 64 in FIG. 2) of the transmitting board 21, and a connecting terminal 24 to be connected to a flexible board 31 by, for example, soldering. The antenna 23 is, as shown in FIG. 2, configured in a coil form on a rear surface of the transmitting board 21. The radio transmitting unit 20 extracts a signal having a predetermined frequency, amplitude, and waveform, from mixing signals generated by the chip component 92 (semiconductor member), by the oscillating circuit 22, and transmits the extracted signal from the antenna 23 to the outside of the capsule endoscope 3.

The illuminating board 71, the imaging board 81, the switching board 11, the power board 12, and the transmitting board 21 are made of rigid boards. As shown in FIG. 3, each of the rigid boards is arranged in a manner for sandwiching the flexible board 31 and structures a rigid/flexible-printed circuit board 32. In other words, each of the rigid boards of the illuminating board 71, the imaging board 81, the switching board 11, the power board 12, and the transmitting board 21, is arranged in that order with a predetermined space via the flexible board 31 and each of which is electrically connected. By folding the flexible board 31 of the rigid/flexible-printed circuit board 32, the illuminating board 71, the imaging board 81, the switching board 11, the power board 12, and the transmitting board 21, are laminated in a cross direction from the side of the front-tip cover 61 to the rear tip portion 64.

As described, according to the present invention, because the stair-shaped LED is arranged along a direction of a circumference "A" having a radius that is wider than that of the field of view determined by the optical quality of the imaging lens 83, with the imaging lens 83 of the imaging unit 8 as a center, the distance between the LED and the front-tip cover gets longer, and therefore, the capsule endoscope can be downsized and the optical flare can hardly be generated.

### Second embodiment

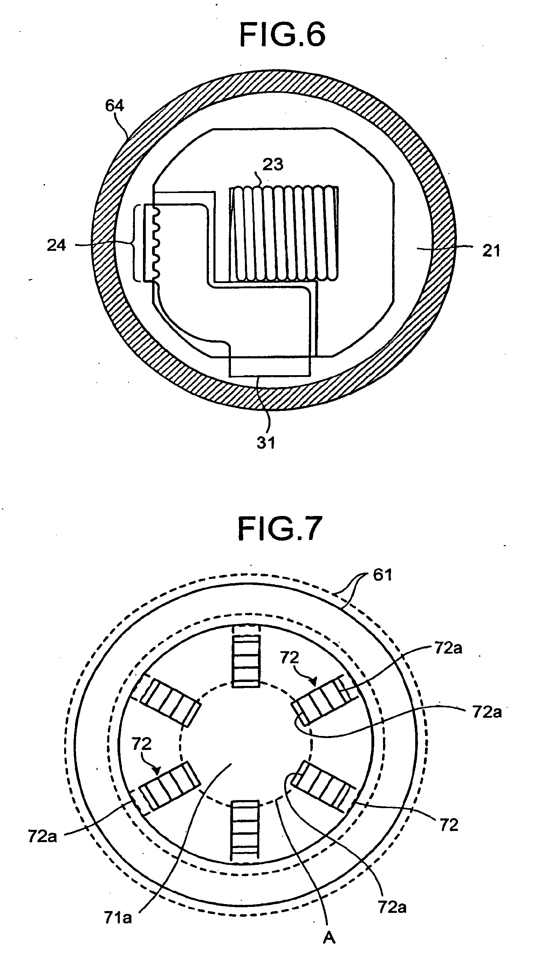

FIG. 7 is a sectional view of a front surface of the illuminating board shown in FIG. 2, according to a second embodiment of the present invention. In the drawings described below, for a convenience of the description, the same reference numeral will be assigned to the same components described in the first embodiment.

In FIG. 7, the present embodiment is different from the first embodiment in that the stair-shaped LED 72 is arranged so that a longitudinal side is to be along a direction of a radius having a width wider than that of the field of view determined by the optical quality of the imaging lens 83, with the imaging lens 83 of the imaging unit 8 as a center, in an equally-spaced manner. A view angle θ of the field of view is determined as, for example, approximately 120° to 130°.

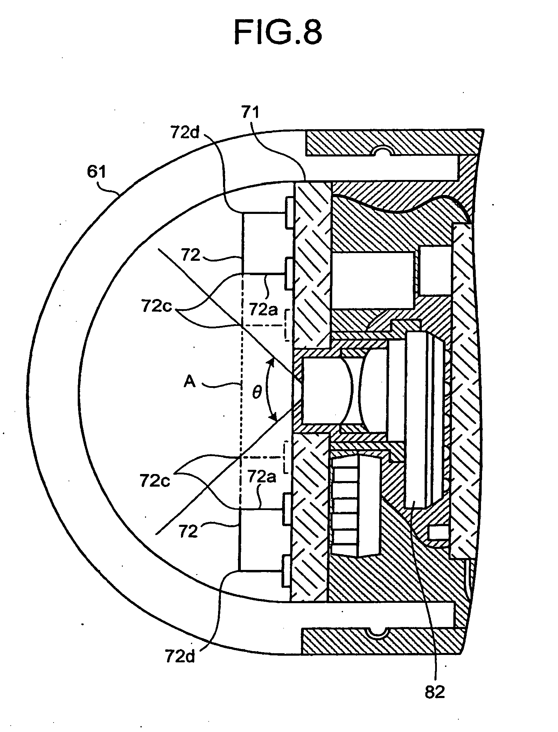

According to the present embodiment, as shown in FIG. 8, if the LED 72 is arranged on a position to which the LED 72 is shifted so that an inner corner portion 72c comes contact with the circumference "A" having a slightly wider width than that of the field of view (a dashed line portion), a longer distance compared to using the conventional LED is generated between an outer corner portion 72d and the inner surface of the front-tip cover 61. Accordingly, the illuminating board 71 can be downsized by a corresponding distance. As a result, the front-tip cover 61 can be downsized and the entire capsule endoscope can also be downsized.

Further, if the LED 72 according to the present embodiment is arranged on a position shifted toward the inner surface of the front-tip cover 61 (a solid line portion), the LED 72 can be arranged on the circumference having a much wider width than that of the field of view, with an enough space, and the optical flare can be prevented from being generated. In this case, because a distance between each of the LEDs 72 becomes wider, it is possible to increase the number of the LEDs 72 to be arranged. Similar to the first embodiment, if the arrangement position of the LED 72 is adjusted within an area corresponding to the distance generated between the LED 72 and the front-tip cover 61, it becomes possible to downsize the front-tip cover 61, prevent the optical flare from being generated, and increase the number of the LEDs 72.

As described, according to the present embodiment, because each of the stair-shaped LEDs is arranged along a direction of a radius having a wider width than that of the field of view determined by the optical quality of the imaging lens 83, with the imaging lens 83 of the imaging unit 8 as a center, the distance between the LED and the front-tip cover gets longer, similar to the first embodiment. As a result, the capsule endoscope can be downsized and the optical flare can hardly be generated.

### Third embodiment

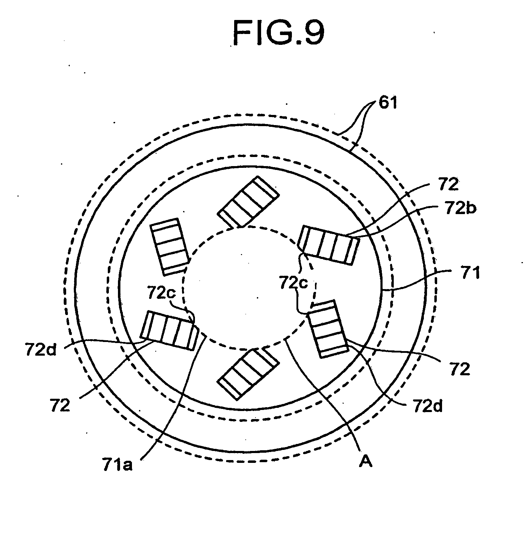

FIG. 9 is a sectional view of a front surface of an illuminating board shown in FIG. 2, according to a third embodiment of the present invention. In the drawing, the present embodiment is different from the second embodiment in that the stair-shaped LED 72 is arranged with a longitudinal side being configured to be in a spiral manner inclined from a direction of a radius having a wider width than the field of view determined by the optical quality of the imaging lens 83, with the imaging lens 83 of the imaging unit 8 as a center.

According to the present embodiment, if the LED 72 is arranged on a position shifted so that the inner corner portion 72c of the LED 72, which is inclined by 45° from the radial direction, comes contact with the circumference "A" having the slightly wider width than that of the field of view, the distance between the outer corner portion 72d and the front-tip cover 61 becomes longer than the one described in the second embodiment. Accordingly, the illuminating board 71 can be downsized by a corresponding distance. As a result, the front-tip cover 61 can be downsized and the entire capsule endoscope can also be downsized.

Further, if the LED 72 according to the present embodiment is arranged on a position shifted toward the inner surface of the front-tip cover 61, the LED 72 can be arranged on the circumference having the wider width than that of the field of view, with an enough space, and the optical flare can be prevented from being generated. In this case, because a distance between each of the LEDs 72 becomes wider, it is possible to increase the number of the LEDs 72 to be arranged. Similar to the first embodiment, if the arrangement position of the LED 72 is adjusted in an area corresponding to the distance generated between the LED 72 and the front-tip cover 61, it becomes possible to downsize the front-tip cover 61, prevent the optical flare from being generated, and increase the number of the LEDs 72.

As described, according to the present embodiment, because each of the stair-shaped LEDs 72 is arranged in a spiral manner inclined from a direction of a radius having a wider width than that of the field of view determined by the optical quality of the imaging lens 83, with the imaging lens 83 of the imaging unit 8 as a center, the distance between the LED and the front-tip cover gets longer. As a result, similar to the first embodiment, the capsule endoscope can be downsized and the optical flare can hardly be generated.

### INDUSTRIAL APPLICABILITY

As described above, the body insertable apparatus according to an embodiment of the present invention is suitable for a medical observation apparatus that observes an examined region, by being inserted inside the human body. Specifically, the body insertable apparatus is suitable for preventing the optical flare from being generated and downsizing the capsule endoscope.

## Claims

1. A body insertable apparatus (3) comprising:
an outer case (6) having at least one dome-shaped end portion;
an imaging unit (8), arranged in the outer case (6), that captures an image of an inside of a body (1) into which the body insertable apparatus (3) being inserted, and acquires an image information of the inside of the body (1);
an illuminating unit (7) that includes an electrode unit (72a) including two electrodes and provided in the dome-shaped end portion and in the periphery of the imaging unit (8), and an irradiation unit (72b) formed on a top portion of the electrode unit (72a) including the two electrodes, the irradiation unit (72b) providing a plurality of light emitting diodes (72) that outputs an illuminating light for illuminating the inside of the body (1) from which an image is captured by the imaging unit (8); and
an arrangement board (71), provided in the outer case (6), on which the imaging unit (8) and the illuminating unit (7) are arranged respectively,
whereby
a distance between end surfaces (72b1) of the irradiation unit (72b) is configured to be shorter than a distance between end surfaces (72a1) of the electrode unit (72a), and
the irradiation unit (72b) is formed in a stair shape with the end surface (72b1) of the irradiation unit (72b) in a longitudinal direction being configured to be shorter than the end surface (72a1) of the electrode unit (72a), and
the light emitting diodes (72) are arranged outside a region having a predetermined radius around the imaging unit (8), the predetermined radius being set larger than a field of view determined by an optical quality of an optical system of the imaging unit (8).

2. The body insertable apparatus (3) according to claim 1, wherein each of the light emitting diodes (72) is arranged so that a longitudinal side of the light emitting diode (72) is arranged along a circumferential direction with a predetermined radius having the imaging unit (8) as a center.

3. The body insertable apparatus (3) according to claim 1, wherein each of the light emitting diodes (72) is arranged so that a longitudinal side of the light emitting diode (72) is arranged along a radial direction with a predetermined radius having the imaging unit (8) as a center.

4. The body insertable apparatus (3) according to claim 1, wherein each of the light emitting diodes (72) is arranged so that a longitudinal side of the light emitting diode (72) is arranged being inclined from a radial direction with a predetermined radius having the imaging unit (8) as a center.

5. The body insertable apparatus (3) according to claim 3 or 4, wherein the light emitting diodes (72) are arranged such that an inner corner portion of the light emitting diode (72) is disposed on a substantially conical, imaginary surface whose tip is on the imaging unit (8) and which demarcates the field of view.

6. The body insertable apparatus (3) according to claim 1, wherein the electrode unit (72a) includes two electrodes, and a distance between one longitudinal end surface of one electrode and a surface of the other electrode farthest from the one longitudinal end surface is longer than a distance between longitudinal end surfaces of the irradiation unit (72b).

7. The body insertable apparatus (3) according to claim 6, wherein a ratio of the distance between the end surfaces of the two electrodes to the distance between the longitudinal end surfaces of the irradiation unit (72b) is substantially 1.6:1.

## Patentansprüche

1. In einen Körper einführbares Gerät (3), das umfasst:
hat; ein äußeres Gehäuse (6), das mindestens einen kuppelförmigen Endabschnitt hat;
eine in dem äußeren Gehäuse (6) angeordnete Bildgebungseinheit (8), die ein Bild eines Inneren eines Körpers (1) aufnimmt, in den das in einen Körper einführbare Gerät (3) eingeführt ist, und die eine Bildinformation von dem Inneren des Körpers (1) erfasst;
eine Beleuchtungseinheit (7), die eine Elektrodeneinheit (72a), die zwei Elektroden umfasst und in dem kuppelförmigen Endabschnitt und in dem Umfang der Bildgebungseinheit (8) vorgesehen ist, und eine Bestrahlungseinheit (72b) umfasst, die an einem oberen Abschnitt der die zwei Elektroden umfassenden Elektrodeneinheit (72a) ausgebildet ist, wobei die Bestrahlungseinrichtung (72b) eine Mehrzahl von Licht emittierenden Dioden (72) bereitstellt, die ein Beleuchtungslicht zum Beleuchten des Inneren des Körpers (1) ausgibt, von dem durch die Bildgebungseinheit (8) ein Bild aufgenommen wird; und
eine in dem äußeren Gehäuse (6) vorgesehene Trägerplatine (71), auf der die Bildgebungseinheit (8) und die Beleuchtungseinheit (7) jeweils angeordnet sind, wodurch
ein Abstand zwischen Endoberflächen (72b1) der Bestrahlungseinheit (72b) dazu eingerichtet ist, kürzer als ein Abstand zwischen Endoberflächen (72a1) der Elektrodeneinheit (72a) zu sein, und
die Bestrahlungseinheit (72b) in einer Stufenform ausgebildet ist, wobei die Endoberfläche (72b1) der Bestrahlungseinheit (72b) in einer Längsrichtung dazu eingerichtet ist, kürzer als die Endoberfläche (72a1) der Elektrodeneinheit (72a) zu sein, und
die Licht emittierenden Dioden (72) außerhalb eines Bereichs angeordnet sind, der einen vorbestimmten Radius um die Bildgebungseinheit (8) hat, wobei der vorbestimmte Radius so festgelegt ist, dass er größer ist als ein Beobachtungsbereich, der durch eine optische Qualität eines optischen Systems der Bildgebungseinheit (8) bestimmt wird.

2. In einen Körper einführbares Gerät (3) gemäß Anspruch 1, bei dem jede der Licht emittierenden Dioden (72) so angeordnet ist, dass eine Längsseite der Licht emittierenden Diode (72) entlang einer Umfangsrichtung mit einem vorbestimmten Radius, der die Bildgebungseinheit (8) als Mittelpunkt hat, angeordnet ist.

3. In einen Körper einführbares Gerät (3) gemäß Anspruch 1, bei dem jede der Licht emittierenden Dioden (72) so angeordnet ist, dass eine Längsseite der Licht emittierenden Diode (72) entlang einer radialen Richtung mit einem vorbestimmten Radius, der die Bildgebungseinheit (8) als Mittelpunkt hat, angeordnet ist.

4. In einen Körper einführbares Gerät (3) gemäß Anspruch 1, bei dem jede der Licht emittierenden Dioden (72) so angeordnet ist, dass eine Längsseite der Licht emittierenden Diode (72) geneigt zu einer radialen Richtung mit einem vorbestimmten Radius, der die Bildgebungseinheit (8) als Mittelpunkt hat, angeordnet ist.

5. In einen Körper einführbares Gerät (3) gemäß Anspruch 3 oder 4, bei dem die Licht emittierenden Dioden (72) so angeordnet sind, dass ein innerer Eckabschnitt der Licht emittierende Diode (72) auf einer im Wesentlichen kegelförmigen imaginären Oberfläche angeordnet ist, deren Spitze an der Bildgebungseinheit (8) liegt und die den Beobachtungsbereich abgrenzt.

6. In einen Körper einführbares Gerät (3) gemäß Anspruch 1, bei dem die Elektrodeneinheit (72a) zwei Elektroden umfasst und ein Abstand zwischen einer Längsendoberfläche einer Elektrode und einer Oberfläche der anderen Elektrode, die am weitesten von der einen Längsendoberfläche entfernt ist, länger ist als eine Entfernung zwischen Längsendoberflächen der Bestrahlungseinheit (72b) ist.

7. In einen Körper einführbares Gerät (3) gemäß Anspruch 6, bei dem ein Verhältnis zwischen der Entfernung zwischen den Endoberflächen der zwei Elektroden und der Entfernung zwischen den Längsendoberflächen der Bestrahlungseinheit (72b) im Wesentlichen 1,6:1 ist.

## Revendications

1. Appareil (3) pouvant être inséré dans un corps comprenant :
un boîtier externe (6) comportant au moins une partie d'extrémité en forme de dôme ;
une unité d'imagerie (8), agencée dans le boîtier externe (6), qui capture une image de l'intérieur d'un corps (1) dans lequel l'appareil (3) pouvant être inséré dans un corps est inséré, et acquiert des informations d'image de l'intérieur du corps (1) ;
une unité d'éclairage (7) qui comprend une unité d'électrodes (72a) comprenant deux électrodes et prévue dans la partie d'extrémité en forme de dôme et dans la périphérie de l'unité d'imagerie (8), et une unité d'irradiation (72b) formée sur une partie supérieure de l'unité d'électrodes (72a) incluant les deux électrodes, l'unité d'irradiation (72b) fournissant une pluralité de diodes à émission de lumière (72) qui délivrent en sortie une lumière d'éclairage destinée à éclairer l'intérieur du corps (1) à partir duquel une image est capturée par l'unité d'imagerie (8) ; et
un panneau d'agencement (71), prévu dans le boîtier externe (6), sur lequel l'unité d'imagerie (8) et l'unité d'éclairage (7) sont agencées respectivement, d'où il résulte qu'une distance entre les surfaces d'extrémité (72b1) de l'unité d'irradiation (72b) est configurée pour être plus courte qu'une distance entre les surfaces d'extrémité (72a1) de l'unité d'électrodes (72a), et
l'unité d'irradiation (72b) est formée en forme de marche, la surface d'extrémité (72b1) de l'unité d'irradiation (72b) dans une direction longitudinale étant configurée pour être plus courte que la surface d'extrémité (72a1) de l'unité d'électrodes (72a), et
les diodes à émission de lumière (72) sont agencées à l'extérieur d'une région présentant un rayon prédéterminé autour de l'unité d'imagerie (8), le rayon prédéterminé étant établi plus grand qu'un champ de vision déterminé par une qualité optique d'un système optique de l'unité d'imagerie (8).

2. Appareil (3) pouvant être inséré dans un corps selon la revendication 1, dans lequel chacune des diodes à émission de lumière (72) est agencée de sorte qu'un côté longitudinal de la diode à émission de lumière (72) est agencé le long d'une direction circonférentielle avec un rayon prédéterminé comportant l'unité d'imagerie (8) comme centre.

3. Appareil (3) pouvant être inséré dans un corps selon la revendication 1, dans lequel chacune des diodes à émission de lumière (72) est agencée de sorte qu'un côté longitudinal de la diode à émission de lumière (72) est agencé le long d'une direction radiale avec un rayon prédéterminé comportant l'unité d'imagerie (8) comme centre.

4. Appareil (3) pouvant être inséré dans un corps selon la revendication 1, dans lequel chacune des diodes à émission de lumière (72) est agencée de sorte qu'un côté longitudinal de la diode à émission de lumière (72) est agencé en étant incliné par rapport à une direction radiale avec un rayon prédéterminé comportant l'unité d'imagerie (8) comme centre.

5. Appareil (3) pouvant être inséré dans un corps selon la revendication 3 ou 4, dans lequel les diodes à émission de lumière (72) sont agencées d'une manière telle qu'une partie de coin interne de la diode à émission de lumière (72) est disposée sur une surface imaginaire sensiblement conique dont la pointe se situe sur l'unité d'imagerie (8) et qui délimite le champ de vision.

6. Appareil (3) pouvant être inséré dans un corps selon la revendication 1, dans lequel l'unité d'électrodes (72a) comprend deux électrodes, et une distance entre une surface d'extrémité longitudinale d'une électrode et une surface de l'autre électrode la plus éloignée de la surface d'extrémité longitudinale est plus longue qu'une distance entre les surfaces d'extrémité longitudinales de l'unité d'irradiation (72b).

7. Appareil (3) pouvant être inséré dans un corps selon la revendication 6, dans lequel un rapport de la distance entre les surfaces d'extrémité des deux électrodes par rapport à la distance entre les surfaces d'extrémité longitudinales de l'unité d'irradiation (72b) est sensiblement de 1,6:1.
